(19) Europäisches Patentamt
European Patent Office
Office européen des brevets

(11) Veröffentlichungsnummer : **0 356 973 B1**

(12) # EUROPÄISCHE PATENTSCHRIFT

(45) Veröffentlichungstag der Patentschrift : **11.11.92 Patentblatt 92/46**

(51) Int. Cl.[5] : **C07D 233/34,** C07D 239/10, C07D 243/04, C07D 249/02

(21) Anmeldenummer : **89115871.9**

(22) Anmeldetag : **29.08.89**

(54) **Verfahren zur Herstellung von cyclischen N,N'-Dimethylharnstoffen.**

(30) Priorität : **02.09.88 DE 3829848**

(43) Veröffentlichungstag der Anmeldung : **07.03.90 Patentblatt 90/10**

(45) Bekanntmachung des Hinweises auf die Patenterteilung : **11.11.92 Patentblatt 92/46**

(84) Benannte Vertragsstaaten : **DE FR GB IT**

(56) Entgegenhaltungen :
**EP-A- 0 215 964**
**EP-A- 0 222 664**
**EP-A- 0 249 136**

(73) Patentinhaber : **BASF Aktiengesellschaft**
**Carl-Bosch-Strasse 38**
**W-6700 Ludwigshafen (DE)**

(72) Erfinder : **Betz, Rainer, Dr.**
**Wallstrasse 34**
**W-6700 Ludwigshafen (DE)**
Erfinder : **Hahn, Erwin, Dr.**
**Am Buechsenackerhang 31**
**F-6900 Heidelberg (DE)**
Erfinder : **Fikentscher, Rolf, Dr.**
**Von-Stephan-Strasse 27**
**W-6700 Ludwigshafen (DE)**

**EP 0 356 973 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von cyclischen N,N'-Dimethylharnstoffen der allgemeinen Formel I

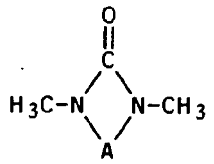 I

in der A eine 1,2- oder 1,3-Alkylengruppe mit 2 bis 10 C-Atomen bedeutet, durch Umsetzung eines cyclischen Harnstoffs der allgemeinen Formel II

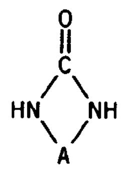 II

mit Formaldehyd und überschüssiger Ameisensäure und anschließende Entfernung der im Reaktionsgemisch noch enthaltenen Ameisensäure.

Für die beispielsweise als Speziallösemittel Verwendung findenden cyclischen N,N'-Dimethylharnstoffe I und insbesondere für die beiden wichtigsten Vertreter dieser Substanzklasse, nämlich N,N'-Dimethyl-1,2-ethylenharnstoff und N,N' -Dimethyl-1,3-propylenharnstoff (engl. Dimethylethyleneurea "DMEU" bzw. Dimethylpropyleneurea "DMPU"), werden in der Literatur zahlreiche Synthesen beschrieben. EP-A- 215 964 beschreibet die Umsetzung eines cyclischen Harnstoffs mit Formaldehyd in Gegenwart von Wasserstoff und einem Hydrierkatalysator.

So betreffen zum Beispiel die Literaturstellen Journal of Chemical and Engineering Data Vol. 21, Nr. 2, 1976, S. 150 (1) und Journal of Organometallic Chemistry Vol. 117, 1976, S. 153 (2) die Herstellung von DMEU und DMPU durch Umsetzung der entsprechenden cyclischen Harnstoffe II mit Formaldehyd und überschüssiger Ameisensäure. Die Schwierigkeit bei der Aufarbeitung der Reaktionsansätze liegt in der Abtrennung der noch vorhandenen Ameisensäure. Aufgrund der hohen Polarität der methylierten Harnstoffe I gelingt es nicht, die ebenfalls stark polare Ameisensäure destillativ von I abzutrennen. Deshalb lehrt (1) die Neutralisation der Ameisensäure mit NaOH zu nicht flüchtigem Natriumformiat und die nachfolgende Destillation von I. (2) gibt die Lehre, I mit HCl in das nicht flüchtige Hydrochlorid zu überführen, danach die Ameisensäure zu verdampfen, den Rückstand durch NaOH wieder in den freien methylierten Harnstoff I umzuwandeln und diesen durch Extraktion und Destillation zu isolieren. Beides sind sehr umständliche Methoden der Aufarbeitung, zumal bei (1) das anfallende Produkt noch durch weitere Reinigungsschritte gesäubert werden muß.

Aus der Angewandten Chemie, 82. Jahrg., 1970, S. 73-77 (3) sind 3:1- und 2:1-Additionsverbindungen von Ameisensäure an tertiäre Amine (III) bekannt. Diese Verbindungen zersetzen sich als isolierte Reinsubstanzen thermisch in Gegenwart von beispielsweise Kupfer(I)-chlorid zu den Aminen III und den gasförmigen Produkten CO, $CO_2$, $H_2$ und/oder $H_2O$.

Der Erfindung lag die Aufgabe zugrunde, den geschilderten Nachteilen bei der Herstellung von I aus II, Formaldehyd und Ameisensäure abzuhelfen.

Demgemäß wurde ein Verfahren zur Herstellung von cyclischen N,N'-Dimethylharnstoffen I

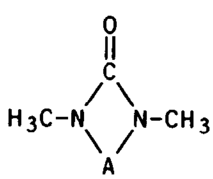 I

in denen A eine 1,2- oder 1,3-Alkylengruppe mit 2 bis 10 C-Atomen bedeutet, durch Umsetzung eines cyclischen Harnstoffes II

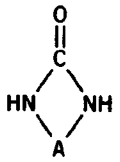

II

mit Formaldehyd und überschüssiger Ameisensäure und anschließende Entfernung der im Reaktionsgemisch noch enthaltenen Ameisensäure gefunden, welches dadurch gekennzeichnet ist, daß man die Ameisensäure mittels eines Katalysatorsystems aus einem tertiären Amin (III) und einem Kupfersalz einer thermischen Zersetzungsreaktion unterwirft.

Die reduktive Alkylierung der cyclischen Harnstoffe II verläuft vermutlich über die Stufe von intermediär gebildeten N-Methylolverbindungen durch Reaktion von II mit Formaldehyd. Man benötigt die Ameisensäure sowohl als Reduktionsmittel für diese Methylolderivate als auch als Lösungsmittel für die Gesamtreaktion. Der Überschuß an Ameisensäure beträgt üblicherweise die 5- bis 10-fache, vorzugsweise die 5- bis 7-fache molare Menge von II. Die verwendete Ameisensäure kann noch bis zu 20 Gew.-% Wasser enthalten.

Der Formaldehyd wird in der Regel als 30 bis 50 gew.-%ige wäßrige Lösung eingesetzt. Es können jedoch auch unter den Reaktionsbedingungen Formaldehyd abgebende Stoffe wie Paraformaldehyd oder Trioxan verwendet werden. Der Formaldehyd wird im allgemeinen in der stöchiometrischen Menge (2 mol pro Mol II) oder vorzugsweise im geringen Überschuß, beispielsweise bis zur 3fachen molaren Menge bezogen auf II, eingesetzt.

Die Umsetzung der cyclischen Harnstoffe II mit Formaldehyd und Ameisensäure erfolgt üblicherweise bei Temperaturen in der Nähe des Siedepunktes der Ameisensäure (101°C), vorzugsweise im Bereich von 80 bis 110°C. Die Reaktionsdauer beträgt in der Regel 12 bis 20 Stunden.

In einer bevorzugten Ausführungsform des erfindungsgemäßen Verfahrens destilliert man einen Teil der im Reaktionsgemisch nach erfolgter Umsetzung noch vorhandenen Ameisensäure zusammen mit Wasser ab, wobei die Destillation bei vermindertem Druck oder vorzugsweise bei Normaldruck erfolgt, wonach man die zurückbleibende Menge der thermischen Zersetzungsreaktion unterwirft. Die Restmenge an Ameisensäure im Destillationsrückstand liegt normalerweise im Bereich von 10 bis 30 % der für die Umsetzung eingesetzten Menge. Arbeitet man bei Temperaturen unterhalb von etwa 115°C, kann die Zugabe des Katalysatorsystems aus dem Amin III und dem Kupfersalz zu jedem Zeitpunkt des Verfahrens vor der Zersetzungsreaktion der Ameisensäure erfolgen, weil das Katalysatorsystem erst bei den Temperaturen der Zersetzungsreaktion wirksam wird. Nach der Zersetzungsreaktion wird das Produkt I durch Destillation isoliert.

Bei einer weiteren bevorzugten Ausführungsform des erfindungsgemäßen Verfahrens destilliert man zunächst bei vermindertem Druck oder vorzugsweise bei Normaldruck ein Ameisensäure-Wasser-Gemisch und danach bei vermindertem Druck, vorzugsweise bei 5 bis 100 mbar, insbesondere bei 10 bis 50 mbar, ein Gemisch aus Ameisensäure und dem Produkt I ab. Anschließend wird das abdestillierte Gemisch aus Ameisensäure und I, welches im allgemeinen noch 10 bis 30 % der für die Umsetzung eingesetzten Ameisensäuremenge enthält, mit dem Katalysatorsystem aus dem Amin III und dem Kupfersalz versetzt und der thermischen Zersetzungsreaktion unterworfen. Nach der Zersetzungsreaktion wird das Produkt I durch Destillation isoliert.

Als Katalysator für die Zersetzung der intermediär aus Ameisensäure und tertiären Amin (III) gebildeten Additionsverbindungen kommen Kupfersalze, vorzugsweise Kupfer(I)-halogenide, insbesondere Kupfer(I)-chlorid, in Betracht. Die Kupfersalze werden vorzugsweise in einer Menge von 0,1 bis 5 mol-%, besonders von 0,5 bis 2 mol-%, pro Mol der zu zersetzenden Ameisensäure verwendet.

Als tertiäre Amine (III) sind die in (3) aufgeführten Amine zu nennen. Bevorzugt werden Trialkylamine, deren Alkylgruppen gleich oder verschieden sein können und jeweils 1 bis 4 C-Atome enthalten. Als Beispiele für Trialkylamine sind anzuführen: Trimethylamin, Triethylamin, Tri-n-propylamin, Triisopropylamin, Tri-n-butylamin, Triisobutylamin, Tri-sec-butylamin, Tri-tert-butylamin, Dimethylethylamin, Diethylmethylamin, Diisopropylmethylamin und Diisopropylethylamin. Besonders bevorzugt wird Triethylamin. Weiterhin können aber auch cyclische Amine wie N-Methylpiperidin, N-Methylpyrrolidin, N-Methylmorpholin oder 1,4-Diazabicyclo[2, 2, 2]octan oder araliphatische Amine wie N,N-Dimethylbenzylamin verwendet werden. Man kann auch Mischungen der genannten Amine einsetzen. Die tertiären Amine (III) werden vorzugsweise in einer Menge von 0,1 bis 5 mol-% besonders von 0, 5 bis 2 mol -%, pro Mol der zu zersetzenden Ameisensäure verwendet.

Die thermische Zersetzungsreaktion der Ameisensäure läuft mit dem erfindungsgemäßen Katalysatorsystem im Temperaturbereich von 120 bis 250°C, vorzugsweise von 140 bis 200°C, insbesondere von 150 bis 170°C, ab. Das Einsetzen der Zersetzungsreaktion macht sich durch eine lebhafte Gasentwicklung bemerkbar. Die Dauer der Zersetzungsreaktion beträgt normalerweise 4 bis 10 Stunden.

Das erfindungsgemäße Verfahren läßt sich vorteilhaft auf die Herstellung der Fünf- und Sechsring-Dimethylharnstoffe I anwenden. Als Beispiele für I sind anzuführen:

N,N'-Dimethyl-1,2-ethylenharnstoff (DMEU), N,N'-Dimethyl-1,2-propylenharnstoff, N,N'-Dimethyl-1,3-propylenharnstoff (DMPU), N,N'-Dimethyl-1,2-butylenharnstoff, N,N'-Dimethyl-2,3-butylenharnstoff, N,N'-Dimethyl-1,3-butylenharnstoff, 4,5-Diethyl-1,3-dimethyl-2-imidazolidinon und 4,6-Diethyl-1,3-dimethyl-3,4,5,6-tetrahydro-2(1H)-pyrimidinon. Besondere Bedeutung hat das Verfahren für die Herstellung von DMEU und DMPU.

Das erfindungsgemäße Verfahren stellt eine einfache und effektive Methode zur Entfernung von Ameisensäure aus den Reaktionsansätzen der Umsetzung der cyclischen Harnstoffe II zu den methylierten Verbindungen I dar. Insbesondere treten keine Ausbeuteverluste durch eine umständliche und viele Schritte umfassende Aufarbeitung, wie sie in (1) oder (2) beschrieben ist, auf. Die Ausbeuten beim erfindungsgemäßen Verfahren liegen somit deutlich höher.

Beispiel 1

Herstellung von N,N'-Dimethyl-1,3-propylenharnstoff (DMPU)

Eine Mischung aus 400 g (4,0 mol) 1,3-Propylenharnstoff, 920 g (20 mol) Ameisensäure (98-100 Gew.-%), 576 g einer 50 gew.-%igen wäßrigen Formaldehyd-Lösung (≙ 9,6 mol Formaldehyd), 4,0 g (40 mmol) Triethylamin und 4,0 g (40 mmol) Kupfer(I)-chlorid wurde 16 Stunden unter Rückfluß erhitzt. Anschließend wurde Ameisensäure bei Normaldruck zusammen mit Wasser abdestilliert, bis der Destillationsrückstand eine Temperatur von 150°C erreicht hatte. Die im Rückstand verbliebene Menge an Ameisensäure betrug 160 g (3,5 mol). Ab 150°C setzte eine lebhafte Gasentwicklung ein. Es wurde 5 bis 10 Stunden bei dieser Temperatur gerührt, wonach die Säurezahl der Reaktionsmischung unter 1 mg KOH/g fiel. Das Produkt wurde durch Destillation bei 60 mbar und einem Siedepunkt von 144-146°C isoliert und war ausreichend sauber. Die Ausbeute an DMPU betrug 94 %.

Beispiel 2

Herstellung von N,N'-Dimethyl-1,2-ethylenharnstoff (DMEU)

Eine Mischung aus 344 g (4,0 mol) 1,2-Ethylenharnstoff, 920 g (20 mol) Ameisensäure (98-100 Gew.-%) und 576 g einer 50 gew.-%gen wäßrigen Formaldehyd-Lösung (≙ 9,6 mol Formaldehyd) wurde 16 Stunden unter Rückfluß erhitzt. Anschließend wurde Ameisensäure bei Normaldruck zusammen mit Wasser abdestilliert, bis der Destillationsrückstand eine Temperatur von 150°C erreicht hatte. Danach wurde das Produkt bei 50 mbar zusammen mit 160 g (3,5 mol) verbliebener Ameisensäure überdestilliert. Dieses Destillat wurde mit 4,0 g (40 mmol) Triethylamin und 4,0 g (40 mmol) Kupfer(I)-chlorid versetzt und bei 150°C 4 bis 6 Stunden unter lebhafter Gasentwicklung gerührt, wonach die Säurezahl der Mischung unter 1 mg KOH/g fiel. Das Produkt wurde durch Destillation bei 23 mbar und einem Siedepunkt von 106-108°C isoliert und war ausreichend sauber. Die Ausbeute an DMEU betrug 80 %.

## Patentansprüche

1. Verfahren zur Herstellung von cyclischen N,N'-Dimethylharnstoffen der allgemeinen Formel I

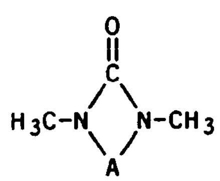

I

in der A eine 1,2- oder 1,3-Alkylengruppe mit 2 bis 10 C-Atomen bedeutet, durch Umsetzung eines cyclischen Harnstoffs der allgemeinen Formel II

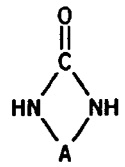

II

mit Formaldehyd und überschüssiger Ameisensäure und anschließende Entfernung der im Reaktionsgemisch noch enthaltenen Ameisensäure, dadurch gekennzeichnet, daß man die Ameisensäure mittels eines Katalysatorsystems aus einem tertiären Amin (III) und einem Kupfersalz einer thermischen Zersetzungsreaktion unterwirft.

2. Verfahren nach Anspruch 1, dadurch gekennzeichnet, daß man einen Teil der im Reaktionsgemisch nach erfolgter Umsetzung noch vorhandenen Ameisensäure abdestilliert und die zurückbleibende Menge der Zersetzungsreaktion unterwirft.

3. Verfahren nach Anspruch 1, dadurch gekennzeichnet, daß man nach erfolgter Umsetzung das gebildete Produkt I zusammen mit noch vorhandener Ameisensäure abdestilliert und die Ameisensäure im Destillat der Zersetzungsreaktion unterwirft.

4. Verfahren nach den Ansprüchen 1 bis 3, dadurch gekennzeichnet, daß man Kupfer(I)-chlorid als Kupfersalz verwendet.

5. Verfahren nach den Ansprüchen 1 bis 4, dadurch gekennzeichnet, daß man als tertiäres Amin (III) ein Trialkylamin verwendet, dessen Alkylgruppen gleich oder verschieden sein können und jeweils 1 bis 4 C-Atome enthalten.

6. Verfahren nach den Ansprüchen 1 bis 5, dadurch gekennzeichnet, daß man das tertiäre Amin (III) und das Kupfersalz in einer Menge von jeweils 0,1 bis 5 mol-% pro Mol der zu zersetzenden Ameisensäure einsetzt.

7. Verfahren nach den Ansprüchen 1 bis 6, dadurch gekennzeichnet, daß man die Zersetzungsreaktion bei Temperaturen von 120 bis 250°C vornimmt.

8. Verfahren nach den Ansprüchen 1 bis 7, dadurch gekennzeichnet, daß man es auf die Herstellung von N,N′-Dimethyl-1,2-ethylen- oder N,N′-Dimethyl-1,3-propylenharnstoff anwendet.

## Claims

1. A process for the preparation of a cyclic N,N′-dimethylurea of the formula I

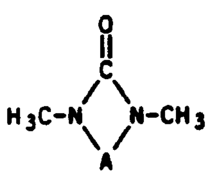

I

where A is a 1,2- or 1,3-alkylene group of 2 to 10 carbon atoms, by reacting a cyclic urea of the formula II

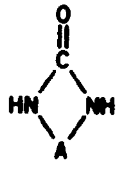

II

with formaldehyde and excess formic acid and then removing the formic acid still present in the reaction mixture, wherein the formic acid is subjected to a thermal decomposition reaction by means of a catalyst system consisting of a tertiary amine (III) and a copper salt.

2. A process as claimed in claim 1, wherein some of the formic acid still present in the reaction mixture after the reaction is distilled off and the remaining amount is subjected to the decomposition reaction.

3. A process as claimed in claim 1, wherein, after the end of the reaction, the product I formed is distilled off together with formic acid still present, and the formic acid in the distillate is subjected to the decomposition reaction.

4. A process as claimed in any of claims 1 to 3, wherein the copper salt used is copper(I) chloride.

5. A process as claimed in any of claims 1 to 4, wherein the tertiary amine (III) used is a trialkylamine whose alkyl groups may be identical or different and are each of 1 to 4 carbon atoms.

6. A process as claimed in any of claims 1 to 5, wherein the tertiary amine (III) and the copper salt are each used in an amount of from 0.1 to 5 mol % per mole of the formic acid to be decomposed.

7. A process as claimed in any of claims 1 to 6, wherein the decomposition reaction is carried out at from 120 to 250°C.

8. A process as claimed in any of claims 1 to 7, which is used for the preparation of N,N′-dimethyl-1,2-ethyleneurea or N,N′-dimethyl-1,3-propyleneurea.

## Revendications

1. Procedé de préparation de N,N′-diméthylurées cycliques de formule générale I

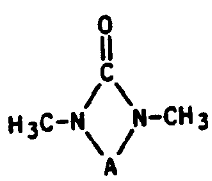

I

dans laquelle A représente un groupement 1,2- ou 1,3-alkylène à 2-10 atomes de carbone, par réaction d'une urée cyclique de formure générale

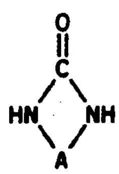

II

avec du formaldéhyde et de l'acide formique en excès, suivie d'élimination de l'acide formique encore contenu dans le mélange réactionnel, caractérisé en ce qu'on soumet l'acide formique à une réaction de décomposition thermique au moyen d'un système catalytique composé d'une amine tertiaire (III) et d'un sel de cuivre.

2. Procédé selon la revendication 1, caractérisé en ce qu'on chasse par distillation une partie de l'acide formique encore présent dans le mélange réactionnel après l'achèvement de la réaction, et on en soumet la quantité restante à la réaction de décomposition.

3. Procédé selon la revendication 1, caractérisé en ce qu'après l'achèvement de la réaction, on chasse par distillation le produit I formé, en même temps que l'acide formique encore présent, et on soumet à la réaction de décomposition l'acide formique contenu dans le distillat.

4. Procédé selon l'une quelconque des revendications 1 à 3, caractérisé en ce qu'on utilise, comme sel de cuivre, du chlorure de cuivre (I).

5. Procédé selon l'une quelconque des revendications 1 à 4, caractérisé en ce qu'on utilise, comme amine tertiaire (III), une trialkylamine dont les groupements alkyle peuvent être identiques ou différents et contiennent chacun de 1 à 4 atomes de carbone.

6. Procédé selon l'une quelconque des revendications 1 à 5, caractérisé en ce qu'on utilise l'amine tertiaire (III) et le sel de cuivre dans une proportion de 0,1 à 5% en moles pour chacun d'entre eux, par mole d'acide formique à décomposer.

7. Procédé selon l'une quelconque des revendications 1 à 6, caractérisé en ce qu'on procède à la réaction de décomposition à des températures de 120 à 250°C.

8. Procédé selon l'une quelconque des revendications 1 à 7, caractérisé en ce qu'il est appliqué à la préparation de N,N'-diméthyl-1,2-éthylène-urée ou de N,N'-diméthyl-1,3-propylène-urée.